(19) Europäisches Patentamt
European Patent Office
Office européen des brevets

(11) EP 1 869 072 B1

# (12) EUROPEAN PATENT SPECIFICATION

(45) Date of publication and mention of the grant of the patent:
**03.10.2012 Bulletin 2012/40**

(21) Application number: **06843768.0**

(22) Date of filing: **27.12.2006**

(51) Int Cl.:
***C07K 14/395*** *(2006.01)*

(86) International application number:
**PCT/JP2006/326399**

(87) International publication number:
**WO 2007/097113 (30.08.2007 Gazette 2007/35)**

## (54) GENE ENCODING PROTEIN RESPONSIBLE FOR FLOCCULATION PROPERTY OF YEAST AND USE THEREOF

GEN, WELCHES FÜR EIN PROTEIN KODIERT, DAS FÜR DIE FLOCKULIERUNGSEIGENSCHAFTEN VON HEFEN VERANTWORTLICH IST, UND DESSEN VERWENDUNG

GÈNE CODANT POUR UNE PROTÉINE RESPONSABLE DE LA PROPRIÉTÉ DE FLOCULATION DE LA LEVURE ET UTILISATION DUDIT GÈNE

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.02.2006 JP 2006049135**

(43) Date of publication of application:
**26.12.2007 Bulletin 2007/52**

(73) Proprietor: **Suntory Holdings Limited**
**Kita-ku, Osaka-shi,**
**Osaka 530-8203 (JP)**

(72) Inventors:
- **NAKAO, Yoshihiro**
  **Mishima-gun, Osaka 618-8503 (JP)**
- **KODAMA, Yukiko**
  **Mishima-gun, Osaka 618-8503 (JP)**
- **SHIMONAGA, Tomoko**
  **Mishima-gun, Osaka 618-8503 (JP)**

(74) Representative: **Vossius & Partner**
**Siebertstrasse 4**
**81675 München (DE)**

(56) References cited:
**EP-A1- 1 236 803**

- **IRMINGER-FINGER I ET AL: "MHP1, AN ESSENTIAL GENE IN SACCHAROMYCES CEREVISIAE REQUIRED FOR MICROTUBULE FUNCTION" THE JOURNAL OF CELL BIOLOGY, ROCKEFELLER UNIVERSITY PRESS, US, vol. 135, 1996, pages 1323-1339, XP000881876 ISSN: 0021-9525**
- **DATABASE EMBL 9 January 2006 (2006-01-09), "Synthetic construct Saccharomyces cerevisiae clone FLH203043.01X MHP1 gene, complete sequence." XP002430010 retrieved from EBI Database accession no. DQ331471**
- **DATABASE EMBL 10 January 2001 (2001-01-10), "T3 end of clone AS0AA023G05 of library AS0AA from strain CLIB 533 of Saccharomyces bayanus." XP002430011 retrieved from EBI Database accession no. AL400785**
- **TOSCH W ET AL: "Molecular species of phosphatidylethanolamine from continuous cultures of Saccharomyces pastorianus syn. carlsbergensis strains" YEAST, vol. 23, no. 2, January 2006 (2006-01), pages 75-82, XP002430007 ISSN: 0749-503X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 869 072 B1

## Description

## TECHNICAL FIELD

The present invention relates to the use of a gene encoding a protein responsible for flocculation property of yeast, in particular, a brewery yeast for producing alcoholic beverages with appropriate flocculation property and a method for producing said beverages. More particularly, the present invention relates to the use of a yeast, which shows appropriate flocculation property by controlling expression level of MHP1 gene encoding a protein responsible for flocculation property of brewery yeast, especially non-ScMHP1 gene specific to a lager brewing yeast in a method for producing alcoholic beverages with said yeast, to a method for selecting said yeast and to a method for breeding said yeast.

## BACKGROUND ART

Flocculation property of yeast used for fermentation in alcoholic beverages is very important. Flocculation property of yeast means property of forming aggregation as a result of interaction among each individual yeast cells, thus sedimenting to the bottom of the liquid in a liquid culture medium.

For example, yeasts used for fermentation of lager-type beer, which is popularly drunk at the present day, are also called bottom fermenting yeast, because the yeast have a character of flocculating and sedimenting to the bottom of fermentation broth near the end of fermentation. In beer brewing, yeast is recovered after fermentation and the recovered yeast is used at the subsequent fermentation, which is called "Renjo" which means successive fermentation. Thus, flocculation property of yeast is very important property from the standpoint of working efficiency of brewing process. That is, yeast having poor flocculation property does not sediment at the end of fermentation, and there is a problem that extraneous steps such as centrifugation are required for recover it. On the other hand, yeast having undesirably high flocculation property may sediment during fermentation, which can result in immature termination of fermentation. In that case, flavor and taste of resultant product are also seriously influenced. Accordingly, it is very important to use yeast having suitable flocculation property for production of desired alcoholic beverages.

Further, flocculation of yeast is actively studied, because efficiency is required especially in the field of industrial alcohol production (Novel agglutinative alcohol fermenting yeast, Japanese Examined Patent Publication (Kokoku) No. H6-36734) and wastewater treatment (Japanese Patent No. 3044284), as well as in the field of alcoholic beverage production.

FLO gene family (FLO1, FLO5, FLO8 FLO9 FLO10, FLO11) and SFL1, etc. have ever been recognized as genes responsible for flocculation property of yeast as a result of enormous quantity of investigations on flocculation property, which is of importance for industry, of yeast as stated above.

In the investigation, for example, property of FLO1 gene was analyzed at molecular level (Bidard et al., YEAST, 11(9), 809, 1995). Method for controlling flocculation property of beer yeast was investigated as well using Flo 1p (Japanese Patent No. 3643404).

Method of controlling flocculation property of yeast by regulating expression of FLO8 gene, and method of judging flocculating property using nucleotide sequence of FLO5 gene (International Publication No. WO01/040514), etc. are also reported.

On the other hand, some of yeast cell surface proteins specific to flocculating yeast are known as well. However, knowledge of each proteins is not sufficient for research to control flocculation property of beer yeast.

## DISCLOSURE OF INVENTION

Under the above situations, there has been a need for breeding yeasts having flocculation property suitable for production of desired alcoholic beverages to make high-efficiency production of alcoholic beverages possible by using a gene encoding a protein responsible for flocculation property of brewery yeast and said protein.

The present inventors made extensive studies to solve the above problems and as a result, succeeded in identifying and isolating a gene encoding a protein responsible for flocculation property of yeast from beer yeast. Moreover, the present inventors produced transformed yeast in which expression of the obtained gene was controlled to verify that flocculation property can be actually controlled, thereby completing the present invention.

Thus, the present invention relates to:

(1) A method for producing an alcoholic beverage by using a yeast, wherein the expression of a polynucleotide is repressed by disrupting the gene according to the polynucleotide wherein the polynucleotide is a polynucleotide encoding a protein having an activity of imparting flocculation property to a yeast selected from the group consisting of:

(a) a polynucleotide comprising a polynucleotide consisting of the nucleotide sequence of SEQ ID NO:1;

(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 in which one to 20 amino acids thereof are deleted, substituted, inserted and/or added, and having an activity of imparting flocculation property to yeast; and
(d) a polynucleotide comprising a polynucleotide encoding a protein having an amino acid sequence having 93% or higher identity with the amino acid sequence of SEQ ID NO:2, and said protein having an activity of imparting flocculation property to yeast.

(2) The method according to (1) above, wherein the flocculation property of said yeast is decreased.

(3) The method according to (1) or (2) above, wherein the brewed alcoholic beverage is a malt beverage.

(4) The method according to (1) or (2) above, wherein the brewed alcoholic beverage is wine.

(5) Use of a polynucleotide as defined in (1) for the preparation of a yeast with an increased flooculation property.

(6) A method for controlling the flocculation property in a yeast comprising the step of controlling the expression of a polynucleotide as defined in (1) in said yeast

(7) A method for increasing the flocculation property in a yeast comprising the step of increasing the expression of a polynucleotide as defined in (1) in said yeast

(8) A method for decreasing the flocculation property in a yeast comprising the step of repressing the expression of a polynucleotide as defined in (1) in said yeast

(9) A method for assessing a test yeast for its flocculation property, comprising using a primer or probe designed based on the nucleotide sequence of a gene having the nucleotide sequence of SEQ ID NO: 1 and encoding a protein having an activity of imparting flocculation property to yeast

(10) A method for assessing a test yeast for its flocculation property, comprising: culturing the test yeast; and measuring the expression level of the gene having the nucleotide sequence of SEQ ID NO: 1 and encoding a protein having an activity of imparting flocculation properly to yeast.

(11) A method for screening a test yeast for an increased flocculation property in comparison to a reference yeast comprising the steps of

(a) culturing a reference yeast and test yeasts;
(b) measuring for each yeast

(i) the expression level of the gene having the nucleotide sequence of SEQ ID NO:1 and encoding a protein having an activity of imparting flocculation property to yeast; or
(ii) the quantity of the protein encoded by a polynucleotide as defined in (1);

wherein an increase in the expression level determined in step (b) (i) or of the quantity of the protein determined in step (b) (ii) in comparison to the reference yeast is indicative for an increased flocculation property in comparison to the reference yeast

(12) A method for screening a yeast for a reduced flocculation property in comparison to a reference yeast comprising the steps of

(a) culturing a reference yeast and test yeasts,
(b) measuring for each yeast

(i) the expression level of the gene having the nucleotide sequence of SEQ ID NO: 1 and encoding a protein having an activity of imparting flocculation property to yeast; or
(i) the quantity of the protein encoded by a polynucleotide as defined in (1) ;

wherein a reduction in the expression level determined in step (b) (i) or of the quantity of the protein determined in step (b) (ii) in comparison to the reference yeast is indicative for a reduced flocculation property in comparison to the reference yeast

14. A method for producing an alcoholic beverage comprising: conducting fermentation for producing an alcoholic beverage using the yeast as defined in (1) or (2), above.

15. A method for producing a yeast in which the flocculation property is decreased characterized by the step of disrupting in said yeast the gene corresponding to the polynucleotide as defined in (1).

According to the method for producing alcoholic beverages using transformed yeast as described herein , alcoholic beverages can be produced highly-efficiently by using yeasts having flocculation property suitable for production of desired alcoholic beverages.

## BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract (sugar) consumption with time upon beer fermentation test. The horizontal axis represents fermentation time while the vertical axis represents apparent extract concentration (w/w%).
Figure 3 shows the expression profile of non-ScMHP1 gene in yeasts upon beer fermentation test. The horizontal axis represents fermentation time while the vertical axis represents the intensity of detected signal.
Figure 4 shows the result of flocculation property test of non-ScMHP1 disrupted strain. The vertical axis represents Segmentation Index indicating flocculation property.

## BEST MODES FOR CARRYING OUT THE INVENTION

The present inventors isolated and identified non-ScMHP1 gene encoding a protein responsible for flocculation property of brewery yeast based on the lager brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2.

### <u>1. Polynucleotide as disclosed herein</u>

The present invention discloses (a) polynucleotide comprising a polynucleotide, of the nucleotide sequence of SEQ ID NO:1; and (b) a polynucleotide comprising a polynucleotide encoding a protein of the amino acid sequence of SEQ ID NO:2. The polynucleotide can be DNA or RNA

The target polynucleotide of the present invention is not limited to the polynucleotide encoding a protein responsible for flocculation property derived from lager brewing yeast described above and may include other polynucleotides encoding proteins having equivalent functions to said protein. Proteins with equivalent functions include, for example, (c) a protein of an amino acid sequence of SEQ ID NO:2 with one to 20 amino acids thereof being deleted, substituted, inserted and/or added and imparting flocculation property to yeast.

Such proteins include a protein consisting of an amino acid sequence of SEQ ID NO:2 with,
1 to 20, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6 (1 to several amino acids), 1 to 5, 1 to 4, 1 to 3, 1 to 2, or 1 amino acid residues thereof being deleted, substituted, inserted and/or added and imparting flocculation property to yeast. In general, the number of deletions, substitutions, insertions, and/or additions is preferably smaller. In addition, such proteins include (d) a protein having an amino acid sequence with 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO:2, and imparting flocculation property to yeast. In general, the percentage identity is preferably higher.

Flocculation property of yeast may be measured, for example, by a method described in Japanese Patent Application Laid-Open No. H8-205890.

Furthermore, the present invention also discloses (e) a polynucleotide comprising a polynucleotide which hybridizes to a polynucleotide consisting of a nucleotide sequence complementary to the nucleotide sequence of SEQ ID NO:1 under stringent conditions and which encodes a protein imparting flocculation property to yeast; and (f) a polynucleotide comprising a polynucleotide which hybridizes to a polynucleotide complementary to a nucleotide sequence of encoding a protein of SEQ ID NO:2 under stringent conditions, and which encodes a protein imparting flocculation property to yeast.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO:1 or polynucleotide encoding the amino acid sequence of SEQ ID NO:2 as a probe. The hybridization method may be a method described, for example, in MOLECULAR CLONING 3rd Ed., CURRENT PROTOCOLS IN MOLECULAR BIOLOGY, John Wiley & Sons 1987-1997, and so on.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Other polynucleotides that can be hybridized include polynucleotides having
93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotide encoding the amino acid sequence of SEQ ID NO:2 as calculated by homology search software, such as FASTA and BLAST using default parameters.

Identity between amino acid sequences or nucleotide sequences may be determined using algorithm BLAST by Karlin and Altschul (Proc. Natl. Acad. Sci. USA, 87: 2264-2268, 1990; Proc. Natl. Acad. Sci. USA, 90: 5873, 1993). Programs called BLASTN and BLASTX based on BLAST algorithm .have been developed (Altschul SF et al., J. Mol. Biol. 215: 403, 1990). When a nucleotide sequence is sequenced using BLASTN, the parameters are, for example, score = 100 and word length = 12. When an amino acid sequence is sequenced using BLASTX, the parameters are, for example, score = 50 and word length = 3. When BLAST and Gapped BLAST programs are used, default parameters for each of the programs are employed.

Also disclosed is (j) a polynucleotide encoding RNA having a nucleotide sequence complementary to a transcript of the polynucleotide (DNA) as defined in (1) above; (k) a polynucleotide encoding RNA that represses the expression of the polynucleotide (DNA) as defined in (1) above through RNAi effect; (1) a polynucleotide encoding RNA having an activity of specifically cleaving a transcript of the polynucleotide (DNA) as defined in (1) above; and (m) a polynucleotide encoding RNA that represses expression of the polynucleotide (DNA) as defined in (1) above through co-supression effect. These polynucleotides may be incorporated into a vector, which can be introduced into a cell for transformation to repress the expression of the polynucleotides (DNA) of (a) to (i) above. Thus, these polynucleotides may suitably be used when repression of the expression of the above DNA is preferable.

The phrase "polynucleotide encoding RNA having a nucleotide sequence complementary to the transcript of DNA" as used herein refers to so-called antisense DNA. Antisense technique is known as a method for repressing expression of a particular endogenous gene, and is described in various publications (see e.g., Hirajima and Inoue: New Biochemistry Experiment Course 2 Nucleic Acids IV Gene Replication and Expression (Japanese Biochemical Society Ed., Tokyo Kagaku Dozin Co., Ltd.) pp.319-347, 1993). The sequence of antisense DNA is preferably complementary to all or part of the endogenous gene, but may not be completely complementary as long as it can effectively repress the expression of the gene. The transcribed RNA has preferably 90% or higher, and more preferably 95% or higher complementarity to the transcript of the target gene. The length of the antisense DNA is at least 15 bases or more, preferably 100 bases or more, and more preferably 500 bases or more.

The phrase "polynucleotide encoding RNA that represses DNA expression through RNAi effect" as used herein refers to a polynucleotide for repressing expression of an endogenous gene through RNA interference (RNAi). The term "RNAi" refers to a phenomenon where when double-stranded RNA having a sequence identical or similar to the target gene sequence is introduced into a cell, the expressions of both the introduced foreign gene and the target endogenous gene are repressed. RNA as used herein includes, for example, double-stranded RNA that causes RNA interference of 21 to 25 base length, for example, dsRNA (double strand RNA), siRNA (small interfering RNA) or shRNA (short hairpin RNA). Such RNA may be locally delivered to a desired site with a delivery system such as liposome, or a vector that generates the double-stranded RNA described above may be used for local expression thereof Methods for producing or using such double-stranded RNA (dsRNA, siRNA or shRNA) are known from many publications (see, e.g., Japanese National Phase PCT Laid-open Patent Publication No. 2002-516062; US 2002/086356A; Nature Genetics, 24(2), 180-183,2000 Feb.; Genesis, 26(4), 240-244, 2000 April; Nature, 407:6802, 319-20, 2002 Sep. 21; Genes & Dev., Vol. 16, (8), 948-958, 2002 Apr. 15; Proc. NatL Acad. Sci. USA., 99(8), 5515-5520, 2002 Apr. 16; Science, 296(5567), 550-553, 2002 Apr. 19; Proc Natl. Acad. Sci. USA, 99:9, 6047-6052, 2002 Apr. 30; Nature Biotechnology, Vol20 (5), 497-500, 2002 May; Nature Biotechnology, Vol. 20(5), 500-505, 2002 May; Nucleic Acids Res., 30:10, e46,2002 May 15).

The phrase "polynucleotide encoding RNA having an activity of specifically cleaving transcript of DNA" as used herein generally refers to a ribozyme. Ribozyme is an RNA molecule with a catalytic activity that cleaves a transcript of a target DNA and inhibits the function of that gene. Design of ribozymes can be found in various known publications (see, e.g., FEBS Lett. 228: 228, 1988; FEBS Lett. 239: 285, 1988; Nucl. Acids. Res. 17: 7059, 1989; Nature 323: 349, 1986; NucL Acids. Res. 19: 6751, 1991; Protein Eng 3: 733, 1990; NucL Acids Res. 19: 3875, 1991; Nucl. Acids Res. 19: 5125, 1991; Biochem Biophys Res Commun 186: 1271, 1992). In addition, the phrase "polynucleotide encoding RNA that represses DNA expression through co-supression effect" refers to a nucleotide that inhibits functions of target DNA by "co-supression".

The term "co-supression" as used herein, refers to a phenomenon where when a gene having a sequence identical or similar to a target endogenous gene is transformed into a cell, the expressions of both the introduced foreign gene and the target endogenous gene are repressed. Design of polynucleotides having a co-supression effect can also be found in various publications (see, e.g., Smyth DR: Curr. Biol. 7: R793, 1997, Martienssen R: Curr. Biol. 6: 810, 1996).

## 2. Protein as disclosed herein

Also disclosed are proteins encoded by any of the polynucleotides (a) to (d) above. A preferred protein comprises an amino acid sequence of SEQ ID NO:2 with one to 20 amino acids thereof being deleted, substituted, inserted and/or added, and imparts flocculation property to yeast.

Such protein includes those having an amino acid sequence of SEQ ID NO:2 with amino acid residues thereof of the number mentioned above being deleted, substituted, inserted and/or added and imparting flocculation property to yeast. In addition, such protein includes those having homology as described above with the amino acid sequence of SEQ ID NO:2 and imparting flocculation property to yeast.

Such proteins may be obtained by employing site-directed mutation described, for example, in MOLECULAR CLONING 3rd Ed., CURRENT PROTOCOLS IN MOLECULAR BIOLOGY, Nuc. Acids. Res., 10:6487(1982) Proc. Natl. Acad. Sci. USA 79: 6409 (1982), Gene 34: 315 (1985), Nuc. Acids. Res., 13: 4431 (1985), Proc. Natl. Acad Sci. USA 82: 488 (1985).

Deletion, substitution, insertion and/or addition of one to 20 amino acid residues in an amino acid sequence of the disclosed protein means that one to 20 amino acid residues are deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

## 3. Vector as disclosed herein and yeast transformed with the vector

Also disclosed is a vector comprising the polynucleotide described above. The vector is a vector including any of the polynucleotides described in (a) to (d) above or any of the polynucleotides described in (j) to (m) above. Generally, the vector comprises an expression cassette including as components (x) a promoter that can transcribe in a yeast cell; (y) a polynucleotide described in any of (a) to (i) above that is linked to the promoter in sense or antisense direction; and (z) a signal that functions in the yeast with respect to transcription termination and polyadenylation of RNA molecule.

In order to highly express the protein described above upon brewing alcoholic beverages (e.g., beer) described below, these polynucleotides are introduced in the sense direction to the promoter to promote expression of the polynucleotide (DNA) described in any of (a) to (d) above. Further, in order to repress the above protein of the invention upon brewing alcoholic beverages (e.g., beer) described below, these polynucleotides are introduced in the antisense direction to the promoter to repress the expression of the polynucleotide (DNA) described in any of (a) to (d), above.

In order to repress the above protein of the invention, the polynucleotide may be introduced into vectors such that the polynucleotide of any of the (j) to (m) is to be expressed. According to the present invention, the target gene (DNA) may be disrupted to repress the expression of the DNA described above or the expression of the protein described above. A gene may be disrupted by adding or deleting one or more bases to or from a region involved in expression of the gene product in the target gene, for example, a coding region or a promoter region, or by deleting these regions entirely. Such disruption of gene may be found in known publications (see, e.g., Proc. Natl. Acad. Sci. USA, 76, 4951 (1979), Methods in Enzymology, 101, 202(1983), Japanese Patent Application Laid-Open No.6-253826).

Further, in the present invention, the expression level of a target gene can be controlled by introducing a mutation to a promoter or genetically altering a promoter by homologous recombination. Such mutation introducing method is described in Nucleic Acids Res. 29, 4238-4250 (2001), and such alteration of a promoter is described in, for example, Appl Environ Microbiol.,72, 5266-5273 (2006).

A vector introduced in the yeast may be any of a multicopy type (YEp type), a single copy type (YCp type), or a chromosome integration type (YIp type). For example, YEp24 (J. R Broach et al., EXPERIMENTAL MANIPULATION OF GENE EXPRESSION, Academic Press, New York 83, 1983) is known as a YEp type vector, YCp50 (M. D. Rose et al., Gene 60: 237, 1987) is known as a YCp type vector, and YIp5 (K. Struhl et aL, Proc. Natl. Acad Sci. USA, 76: 1035, 1979) is known as a YIp type vector, all of which are readily available.

Promoters/terminators for adjusting gene expression in yeast may be in any combination as long as they function in the brewery yeast and they are not influenced by constituents in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et aL, EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et aL, Proc. Natl. Acad. Sci. USA, 81, 337 1984) or a cerulenin-resistant gene (fas2m, PDR4) (Junji Inokoshi et aL, Biochemistry, 64, 660, 1992; and Hussain et al., Gene, 101: 149, 1991, respectively) may be used.

A vector constructed as described above is introduced into a host yeast Examples of the host yeast include any yeast that can be used for brewing, for example, brewery yeasts for beer, wine and sake. Specifically, yeasts such as genus *Saccharomyces* may be used. According to the present invention, a lager brewing yeast, for example, *Saccharomyces pastorianus* W34/70, etc., *Saccharomyces carlsbergensis* NCYC453 or NCYC456, etc., or *Saccharomyces cerevisiae* NBRC1951, NBRC1952, NBRC1953 or NBRC1954, etc., may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth. Enzym., 194: 182 (1990)), a spheroplast method (Proc. Natl. Acad Sci. USA, 75: 1929(1978)), a lithium acetate method (J. Bacteriology, 153: 163 (1983)), and methods described in Proc. Natl. Acad. Sci. USA, 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali metal ion, preferably lithium ion at a concentration of about 1 to 2 M. After the cell is left to stand at about 30°C for about 60 minutes, it is left to stand with DNA to be introduced (about 1 to 20 μg) at about 30°C for about another 60 minutes. Polyethyleneglycol, preferably about 4,000 Dalton of polyethyleneglycol, is added to a final concentration of about 20% to 50%: After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

## 4. Method of producing alcoholic beverages according to the present invention and alcoholic beverages produced by the method

A yeast having flocculation property suitable for a target alcoholic beverages can be obtained by introducing the above described vector or DNA fragments described above to a yeast suitable for brewing target alcoholic beverages to control expression level of the gene. Thus, alcoholic beverages can be produced highly-efficiently. That is to say, desired kind of alcoholic beverages can be produced highly-efficiently by controlling (elevating or reducing) flocculation property using yeasts into which the vector or DNA fragment as described herein was introduced described above, yeasts in which expression of the polynucleotide (DNA) described above was regulated (promoted or suppressed) or yeasts selected by the yeast assessment method of the invention described below for fermentation to produce alcoholic beverages. The target alcoholic beverages include, for example, but not limited, to beer, beer-taste beverages such as sparkling liquor (*happoushu*), wine, whisky, sake and the like. Further, alcohol for practical use such as alcohol for fuel is also included among them.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages. Thus, according to the present invention, alcoholic beverages can be produced highly-efficiently using the existing facility without increasing the cost.

## 5. Yeast assessment method of the invention

The present invention relates to a method for assessing a test yeast for its flocculation property by using a primer or a probe designed based on a nucleotide sequence of a gene having the nucleotide sequence of SEQ ID NO: 1 and encoding a protein imparting flocculation property to yeast. General techniques for such assessment method is known and is described in, for example, WO01/040514, Japanese Laid-Open Patent Application No. H8-205900 or the like. This assessment method is described in below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the gene encoding a protein imparting flocculation property to yeast, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product This method allows prediction and assessment of the flocculation property of yeast as determined by whether the molecular weight of the amplified product is a size that contains the DNA molecule of the specific part. In addition, by analyzing the nucleotide sequence of the amplified product, the property may be predicted and/or assessed more precisely.

Moreover, in the present invention, a test yeast is cultured to measure an expression level of the gene encoding a protein imparting flocculation property to yeast having the nucleotide sequence of SEQ ID NO:1 to assess the test yeast for its flocculation property. In this case, the test yeast is cultured and then RNA or a protein resulting from the gene encoding a protein imparting flocculation property to yeast is quantified. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the gene encoding a protein imparting flocculation property to yeast having the nucleotide sequence of SEQ ID NO:1 are measured to select a test yeast with the gene expression level according to the target flocculation property, thereby selecting a yeast favorable for brewing desired alcoholic beverages. In addition, a reference yeast and a test yeast may be cultured so as to measure and compare the expression level of the gene in each of the yeasts, thereby selecting a favorable test yeast. More specifically, for example, a reference yeast and one or more test yeasts are cultured and an expression level of the gene having the nucleotide sequence of SEQ ID NO:1 is measured in each yeast. By selecting a test yeast with the gene expressed higher (i.e., expression level is enhanced) or lower (i. e., expression level is suppressed) than that in the reference yeast, a yeast suitable for brewing alcoholic beverages can be selected.

Alternatively, test yeasts are cultured and a yeast with a high or low flocculation property is selected, thereby selecting a yeast suitable for brewing desired alcoholic beverages.

In these cases, the test yeasts or the reference yeast may be, for example, a yeast introduced with the vector or DNA fragment of the invention, a yeast in which an expression of a polynucleotide (DNA) of the invention has been controlled, an artificially mutated yeast or a naturally mutated yeast. The flocculation property of yeast can be measured by, for example, a method described in Japanese Patent Application Laid-Open No. HB-205890. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (*see, e.g.*, Yasuji Oshima Ed., BIOCHEMISTRY EXPERIMENTS vol. 39, Yeas Molecular Genetic Experiments, pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Saccharomyces* may be used (*e.g., S. pastorianus, S. cerevisiae,* and *S. carlsbergensis*). According to the present invention, a lager brewing yeast, for example, *Saccharomyces pastorianus* W34/70; *Saccharomyces carlsbeigensis* NCYC453 or NCYC456; or *Saccharomyces cerevisiae* NBRC1951, NBRC1952, NBRC1953 or NBRC1954, etc., may be used. Further, wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used. The reference yeast and the test yeasts may be selected from the above yeasts in any combination.

## EXAMPLES

Hereinafter, the present invention will be described in more detail with reference to working examples. The present invention, however, is not limited to the examples described below.

### Example 1: Cloning of Gene Encoding Protein Responsible for Flocculation Property of Yeast (nonScMHP1)

A gene encoding a protein responsible for flocculation property of brewery yeast (nonScMHP1) (SEQ ID NO: 1) was found as a result of a search utilizing the comparison database described in Japanese Patent Application Laid-Open No. 2004-283169. Based on the acquired nucleotide sequence information, primers nonScMHP1_F (SEQ ID NO: 3) and nonScMHP1_R (SEQ ID NO: 4) were designed to amplify the full-length of the gene. PCR was carried out using chromosomal DNA of a genome sequencing strain, Saccharomyces pastorianus Weihenstephan 34/70 (sometimes abbreviated as "W34/79 strain"), as a template to obtain DNA fragments including the fiill-length gene of nonScMHP1.

The nonScMHP1 gene fragments thus obtained were inserted into pCR2.1-TOPO vector (Invitrogen) by TA cloning. The nucleotide sequences of the nonScMHP1 gene were analyzed by Sanger's method (F. Sanger, Science, 214:1215,1981) to confirm the nucleotide sequence.

### Example 2: Analysis of Expression of nonScMHP1 Gene during Beer Fermentation

A beer fermentation test was conducted using a lager brewing yeast, Saccharomyces pastorianus W34/70.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | $12.8 \times 10^6$ cells/mL |

The fermentation liquor was sampled over time, and the time-course changes in amount of yeast cell growth (Fig. 1) and apparent extract concentration (Fig. 2) were observed. Simultaneously, yeast cells were sampled to prepared mRNA, and the prepared mRNA was labeled with biotin and was hybridized to a beer yeast DNA microarray. The signal was detected using the GeneChip Operating system (GCOS; GeneChip Operating Software 1.0, manufactured by Asymetrix Co). Expression pattern of the nonScMHP1 gene is shown in Figure 3. This result confirmed the expression of the nonScMHP1 gene in the general beer fermentation.

**Example 3: Disruption of nonScMHP1 Gene**

Fragments for gene disruption were prepared by PCR using a plasmid containing a drug resistance marker (pAG25(nat1)) as a template in accordance with a method described in literature (Goldstein et al., Yeast, 15, 1541 (1999)). Primers consisting of nonScMHP1_delta_for (SEQ ID NO.5) and nonScMHP1_delta_rv (SEQ ID NO. 6) were used for the PCR primers.

A spore clone (W34/70-2) isolated from lager brewing yeast Saccharomyces pastorianus strain W34/70 was transformed with the fragments for gene disruption prepared as described above. Transformation was carried out according to the method described in Japanese Patent Application Laid-open No. H07-303475, and transformants were selected on YPD plate medium (1% yeast extract, 2% polypeptone, 2% glucose, 2% agar) containing 50 mg/L of nourseothricin.

**Example 4: Evaluation of Flocculation Property of non-ScMHP1 Disrupted Strain**

Flocculation properties of the disrupted strain obtained in Example 3 and the parent strain (W34/70-2 strain) were evaluated by a method described below.

The yeasts were inoculated into 50 mL of glucose CM medium (3% glucose, 1% bacto peptone, 0.5% yeast extract, 0.5% $KH_2PO_4$, 0.2% $MgSO_4.7H_2O$), then cultured statically at 30°C for 2 days.

Whole amount of the culture was transferred to 450 mL of glucose CM medium and cultured statically at 15°C for 4 days. The yeast cells were collected by centrifugation followed washing twice in cold water of 5°C. One point five grams (1.5 g) of the obtained wet yeast cells were accurately weighed, suspended in 20 mL of 0.1M acetate buffer containing 2mM Ca, and temperature equilibrated in an temperature controlled room at 15°C.

Subsequent measurement procedures were performed in a temperature controlled room at 15°C. The yeast suspension mixed for 30 seconds by vortex mixer was poured into a buret, then immediately 2 mL of the suspension was collected as a 0 min sample. After leaving 10 min at rest, 2 mL of the suspension was collected as a 10 min sample.

OD600 of the collected yeast suspensions were measured, respectively, and flocculation properties (Segmentation Index; SI) were calculated by formula below. The results are indicated in Figure 4. The values were averages of measurement of n = 2.

$$SI=(OD_{10min}-OD_{0min})/OD_{0min}*100$$

As indicated in Figure 4, SI = 139 in the disrupted strain shows that flocculation property of yeast was decreased by disruption of non-ScMHP1 by comparison with SI = 244 in the parent strain.

**Example 5: Construction of nonScMHP1-Highly Expressed Strain**

The nonScMHP1/pCR2.1-TOPO described in Example 1 is digested with the restriction enzymes SacI and NotI to prepare a DNA fragment containing the entire length of the protein-encoding region. This fragment is ligated to pYCGPYNot treated with the restriction enzymes SacI and NotI, thereby constructing the nonScMHP1 high expression vector nonScMHP1/pYCGPYNot. pYCGPYNot is a YCp-type yeast expression vector. A gene inserted is highly expressed by the pyruvate kinase gene PYK1 promoter. The geneticin-resistant gene $G418^r$ is included as the selectable marker in the yeast, and the ampicillin-resistant gene $Amp^r$ as the selectable marker in Escherichia coli.

Using the high expression vector prepared by the above method, the strain Saccharomyces pasteurianus Weihenstephaner 34/70 is transformed by the method described in Japanese Patent Application Laid-open No. H07-303475. The transformants are selected on a YPD plate medium (1% yeast extract, 2% polypeptone, 2% glucose and 2% agar) containing 300 mg/L of geneticin.

Flocculation properties of the highly expressed strain obtained by the method described above and the parent strain (W34/70 strain) are evaluated by the same method as Example 4.

**INDUSTRIAL APPLICABILITY**

The method of producing alcoholic beverages of the present invention may allow for highly-efficient production of alcoholic beverages by using a yeast having suitable for production of desired alcoholic beverages, because the flocculation property of yeast during fermentation can be controlled.

## Claims

1. A method for producing an alcoholic beverage by using a yeast, wherein the expression of a polynucleotide is repressed by disrupting the gene according to the polynucleotide wherein the polynucleotide is a polynucleotide encoding a protein having an activity of imparting flocculation property to yeast selected from the group consisting of:

(a) a polynucleotide comprising a polynucleotide consisting of the nucleotide sequence of SEQ ID NO:1;

(b) a polynucleotide comprising a polynudeotide encoding a protein consisting of the amino acid sequence of SEQ ID NO:2;
(c) a polynucleotide comprising a polynucleotide encoding a protein consisting of the amino add sequence of SEQ ID NO:2 in which one to 20 amino acids thereof are deleted, substituted, inserted and/or added, and having an activity of imparting flocculation property to yeast; and
(d) a polynucleotide comprising a polynucleotide encoding a protein having an amino acid sequence having 93% or higher identity with the amino add sequence of SEQ ID NO:2, and said protein having an activity of imparting flocculation property to yeast

**2.** The method according to Claim 1, wherein the flocculation property of said yeast is decreased.

**3.** The method according to Claim 1 or 2, wherein the brewed alcoholic beverage is a malt beverage.

**4.** The method according to Claim 1 or 2, wherein the brewed alcoholic beverage is wine.

**5.** Use of a polynucleotide as defined in claim 1 for the preparation of a yeast with an increased flocculation property.

**6.** A method for controlling the flocculation property in a yeast comprising the step of controlling the expression of a polynucleotide as defined in claim 1 in said yeast

**7.** A method for increasing the flocculation property in a yeast comprising the step of increasing the expression of a polynucleotide as defined in claim 1 in said yeast.

**8.** A method for decreasing the flocculation property in a yeast comprising the step of repressing the expression of a polynucleotide as defined in claim 1 in said yeast.

**9.** A method for assessing a test yeast for its flocculation property, comprising using a primer or probe designed based on the nucleotide sequence of a gene having the nucleotide sequence of SEQ ID NO: 1 and encoding a protein having an activity of imparting flocculation property to yeast.

**10.** A method for assessing a test yeast for its flocculation property, comprising: culturing the test yeast; and measuring the expression level of the gene having the nucleotide sequence of SEQ ID NO: 1 and encoding a protein having an activity of imparting flocculation property to yeast

**11.** A method for screening a test yeast for an increased flocculation property in comparison to a reference yeast comprising the steps of

(a) culturing a reference yeast and test yeasts;
(b) measuring for each yeast

(i) the expression level of the gene having the nucleotide sequence of SEQ ID NO:1 and encoding a protein having an activity of imparting flocculation property to yeast; or
(ii) the quantity of the protein encoded by a polynucleotide as defined in claim 1;

wherein an increase in the expression level determined in step (b) (i) or of the quantity of the protein determined in step (b) (ii) in comparison to the reference yeast is indicative for an increased flocculation property in comparison to the reference yeast

**12.** A method for screening a yeast for a reduced flocculation property in comparison to a reference yeast comprising the steps of

(a) culturing a reference yeast and test yeasts,
(b) measuring for each yeast

(i) the expression level of the gene having the nucleotide sequence of SEQ ID NO: 1 and encoding a protein having an activity of imparting flocculation property to yeast; or
(i) the quantity of the protein encoded by a polynucleotide as defined in claim 1;

wherein a reduction in the expression level determined in step (b) (i) or of the quantity of the protein determined in step (b) (ii) in comparison to the reference yeast is indicative for a reduced flocculation property in comparison to the reference yeast

**13.** A method for producing an alcoholic beverage comprising: conducting fermentation for producing an alcoholic beverage using the yeast as defined in Claim 1 or 2.

**14.** A method for producing a yeast in which the flocculation property is decreased **characterized by** the step of disrupting in said yeast the gene corresponding to the polynucleotide as defined in claim 1.

**Patentansprüche**

**1.** Verfahren zur Herstellung eines alkoholischen Getränks durch Verwendung einer Hefe, wobei die Expression eines Polynucleotids durch Unterbrechung des Gens gemäß dem Polynucleotid unterdrückt ist, wobei das Polynucleotid ein Polynucleotid ist, das ein Protein codiert, das die Aktivität hat, einer Hefe Flockulierungseigenschaften zu verleihen, ausgewählt aus der Gruppe bestehend aus:

(a) einem Polynucleotid umfassend ein Polynucleotid, das aus der Nucleotidsequenz der SEQ ID NO:1 besteht;
(b) einem Polynucleotid umfassend ein Polynucleotid, das ein Protein codiert, das aus der Aminosäuresequenz der SEQ ID NO:2 besteht;
(c) einem Polynucleotid umfassend ein Polynucleotid, das ein Protein codiert, das aus der Aminosäuresequenz der SEQ ID NO:2 besteht, in der eine bis 20 Aminosäuren davon entfernt, ersetzt, eingefügt, und/oder hinzugefügt sind, und wobei das Protein die Aktivität hat, einer Hefe Flockulierungseigenschaft zu verleihen; und
(d) einem Polynucleotid umfassend ein Polynucleotid, das ein Protein codiert, das eine Aminosäuresequenz hat, die eine 93%ige oder höhere Identität mit der Aminosäuresequenz der SEQ ID NO:2 hat, und wobei das Protein die Aktivität hat, einer Hefe Flockulierungseigenschaft zu verleihen.

**2.** Verfahren gemäß Anspruch 1, wobei die Flockulierungseigenschaft der Hefe verringert ist.

**3.** Verfahren gemäß Anspruch 1 oder 2, wobei das gebraute alkoholische Getränk ein Malzgetränk ist.

**4.** Verfahren gemäß Anspruch 1 oder 2, wobei das gebraute alkoholische Getränk Wein ist.

**5.** Verwendung eines Polynucleotids wie in Anspruch 1 definiert zur Herstellung einer Hefe mit einer gesteigerten Flockulierungseigenschaft.

**6.** Verfahren zum Steuern der Flockulierungseigenschaft in einer Hefe umfassend den Schritt der Steuerung der Expression eines Polynucleotids wie in Anspruch 1 definiert in der Hefe.

**7.** Verfahren zur Steigerung der Flockulierungseigenschaft in einer Hefe umfassend den Schritt der Steigerung der Expression eines wie in Anspruch 1 definierten Polynucleotids in der Hefe.

**8.** Verfahren zum Herabsetzen der Flockulierungseigenschaft in einer Hefe umfassend den Schritt der Repression der Expression eines wie in Anspruch 1 definierten Polynucleotids in der Hefe.

**9.** Verfahren zur Bewertung der Flockulierungseigenschaft einer Testhefe, umfassend die Verwendung eines Primers order einer Sonde, der/die entwickelt wurde basierend auf der Nucleotidsequenz eines Gens, das die Nucleotidsequenz von SEQ ID NO:1 hat und das ein Protein codiert, das eine Aktivität hat, einer Hefe Flockulierungseigenschaft zu verleihen.

**10.** Verfahren zur Bewertung der Flockulierungseigenschaft einer Testhefe, umfassend: Züchten der Testhefe; und Bestimmen des Expressionsspiegels des Gens, das die Nucleotidsequenz von SEQ ID NO:1 hat und das ein Protein codiert, das eine Aktivität hat, einer Hefe Flockulierungseigenschaft zu verleihen.

**11.** Verfahren zum Screenen einer Testhefe auf eine verstärkte Flockulierungseigenschaft im Vergleich zu einer Referenzhefe, umfassend die Schritte:

(a) Züchten einer Referenzhefe und von Testhefen;
(b) Messen in jeder Hefe

(i) des Expressionsspiegels des Gens, das die Nucleotidsequenz der SEQ ID NO: 1 hat und das ein Protein codiert, das eine Aktivität hat, einer Hefe Flockulierungseigenschaft zu verleihen; oder
(ii) der Menge an Protein, das durch das wie in Anspruch 1 definierte Polynucleotid codiert wird;

wobei eine Steigerung des Expressionspiegels, wie in Schritt (b) (i) bestimmt, oder der Proteinmenge, wie in Schritt (b) (ii) bestimmt, im Vergleich zu der Referenzhefe auf eine verstärkte Flockulierungseigenschaft im Vergleich zu der Referenzhefe hindeutet.

**12.** Verfahren zum Screenen einer Testhefe auf eine verringerte Flockulierungseigenschaft im Vergleich zu einer Referenzhefe umfassend die Schritte:

(a) Züchten einer Referenzhefe und von Testhefen;
(b) Messen in jeder Hefe

(i) des Expressionsspiegels des Gens, das die Nucleotidsequenz von SEQ ID NO: 1 hat und das ein Protein codiert, das eine Aktivität hat, einer Hefe Flockulierungseigenschaft zu verleihen; oder
(ii) der Menge an Protein, das durch das wie in Anspruch 1 definierte Polynucleotid codiert wird;

wobei eine Abnahme des Expressionspiegels, wie in Schritt (b) (i) bestimmt, oder der Proteinmenge, wie in Schritt (b) (ii) bestimmt, im Vergleich zu der Referenzhefe auf eine verringerte Flockulierungseigenschaft im Vergleich zu der Referenzhefe hindeutet.

**13.** Verfahren zur Herstellung eines alkoholischen Getränks, umfassend: Durchführen einer Fermentation zur Herstellung eines alkoholischen Getränks unter Verwendung einer Hefe wie in Anspruch 1 oder 2 definiert.

**14.** Verfahren zur Herstellung einer Hefe in welcher die Flockulierungseigenschaft herabgesetzt ist, durch den Schritt der Unterbrechung des Gens, das dem wie in Anspruch 1 definierten Polynucleotid entspricht, in der Hefe.

**Revendications**

**1.** Procédé de production d'une boisson alcoolisée en utilisant une levure, dans lequel l'expression d'un polynucléotide est réprimée par disruption du gène correspondant au polynucléotide, le polynucléotide étant un polynucléotide codant pour une protéine ayant comme activité de conférer une propriété de floculation à la levure sélectionné dans le groupe constitué par :

(a) un polynucléotide comprenant un polynucléotide constitué par la séquence de nucléotides de SEQ ID N° : 1 ;
(b) un polynucléotide comprenant un polynucléotide codant pour une protéine constituée par la séquence d'acides aminés de SEQ ID N°:2;
(c) un polynucléotide comprenant un polynucléotide codant pour une protéine constituée par la séquence d'acides aminés de SEQ ID N° : 2 dans laquelle de 1 à 20 acides aminés sont délétés, substitués, insérés et/ou ajoutés, et ayant comme activité de conférer une propriété de floculation à la levure ; et
(d) un polynucléotide comprenant un polynucléotide codant pour une protéine ayant une séquence d'acides aminés présentant une identité de 93 % ou plus avec la séquence d'acides aminés de SEQ ID N° : 2, et ladite protéine ayant comme activité de conférer une propriété de floculation à la levure.

**2.** Procédé selon la revendication 1, dans lequel la propriété de floculation de ladite levure est diminuée.

**3.** Procédé selon les revendications 1 ou 2, dans lequel la boisson alcoolisée brassée est une boisson au malt.

**4.** Procédé selon les revendications 1 ou 2, dans lequel la boisson alcoolisée brassée est du vin.

**5.** Utilisation d'un polynucléotide comme défini dans la revendication 1 pour préparer une levure ayant une propriété de floculation accrue.

6. Procédé de contrôle de la propriété de floculation chez une levure comprenant l'étape de contrôle de l'expression d'un polynucléotide comme défini dans la revendication 1 chez ladite levure.

7. Procédé d'augmentation de la propriété de floculation chez une levure comprenant l'étape d'augmentation de l'expression d'un polynucléotide comme défini dans la revendication 1 chez ladite levure.

8. Procédé de diminution de la propriété de floculation chez une levure comprenant l'étape de répression de l'expression d'un polynucléotide comme défini dans la revendication 1 chez ladite levure.

9. Procédé d'évaluation de la propriété de floculation d'une levure test, comprenant l'utilisation d'une amorce ou d'une sonde conçue sur la base de la séquence de nucléotides d'un gène ayant la séquence de nucléotides de SEQ ID N° : 1 et codant pour une protéine ayant comme activité de conférer une propriété de floculation à la levure.

10. Procédé d'évaluation de la propriété de floculation d'une levure test, comprenant : la culture de la levure test ; et la mesure du niveau d'expression du gène ayant la séquence de nucléotides de SEQ ID N° : 1 et codant pour une protéine ayant comme activité de conférer une propriété de floculation à la levure.

11. Procédé de criblage d'une levure test pour une propriété de floculation accrue comparativement à une levure de référence comprenant les étapes suivantes :

(a) la culture d'une levure de référence et de levures tests ;
(b) la mesure pour chaque levure

(i) du niveau d'expression du gène ayant la séquence de nucléotides de SEQ ID N° : 1 et codant pour une protéine ayant comme activité de conférer une propriété de floculation à la levure ; ou
(ii) de la quantité de protéine codée par un polynucléotide comme défini dans la revendication 1 ;

dans lequel une augmentation du niveau d'expression déterminé à l'étape (b) (i) ou de la quantité de la protéine déterminée à l'étape (b) (ii) comparativement à la levure de référence indique une propriété de floculation accrue comparativement à la levure de référence.

12. Procédé de criblage d'une levure pour identifier une propriété de floculation réduite comparativement à une levure de référence comprenant les étapes suivantes :

(a) la culture d'une levure de référence et de levures tests ;
(b) la mesure pour chaque levure

(i) du niveau d'expression du gène ayant la séquence de nucléotides de SEQ ID N° : 1 et codant pour une protéine ayant comme activité de conférer une propriété de floculation à la levure ; ou
(ii) de la quantité de protéine codée par un polynucléotide comme défini dans la revendication 1 ;

dans lequel une réduction du niveau d'expression déterminé à l'étape (b) (i) ou de la quantité de la protéine déterminée à l'étape (b) (ii) comparativement à la levure de référence indique une propriété de floculation réduite comparativement à la levure de référence.

13. Procédé de production d'une boisson alcoolisée comprenant : la conduite d'une fermentation pour produire une boisson alcoolisée en utilisant la levure comme défini dans les revendications 1 ou 2.

14. Procédé de production d'une levure chez laquelle la propriété de floculation est réduite, **caractérisé par** l'étape de disruption chez ladite levure du gène correspondant au polynucléotide comme défini dans la revendication 1.

FIG. 1

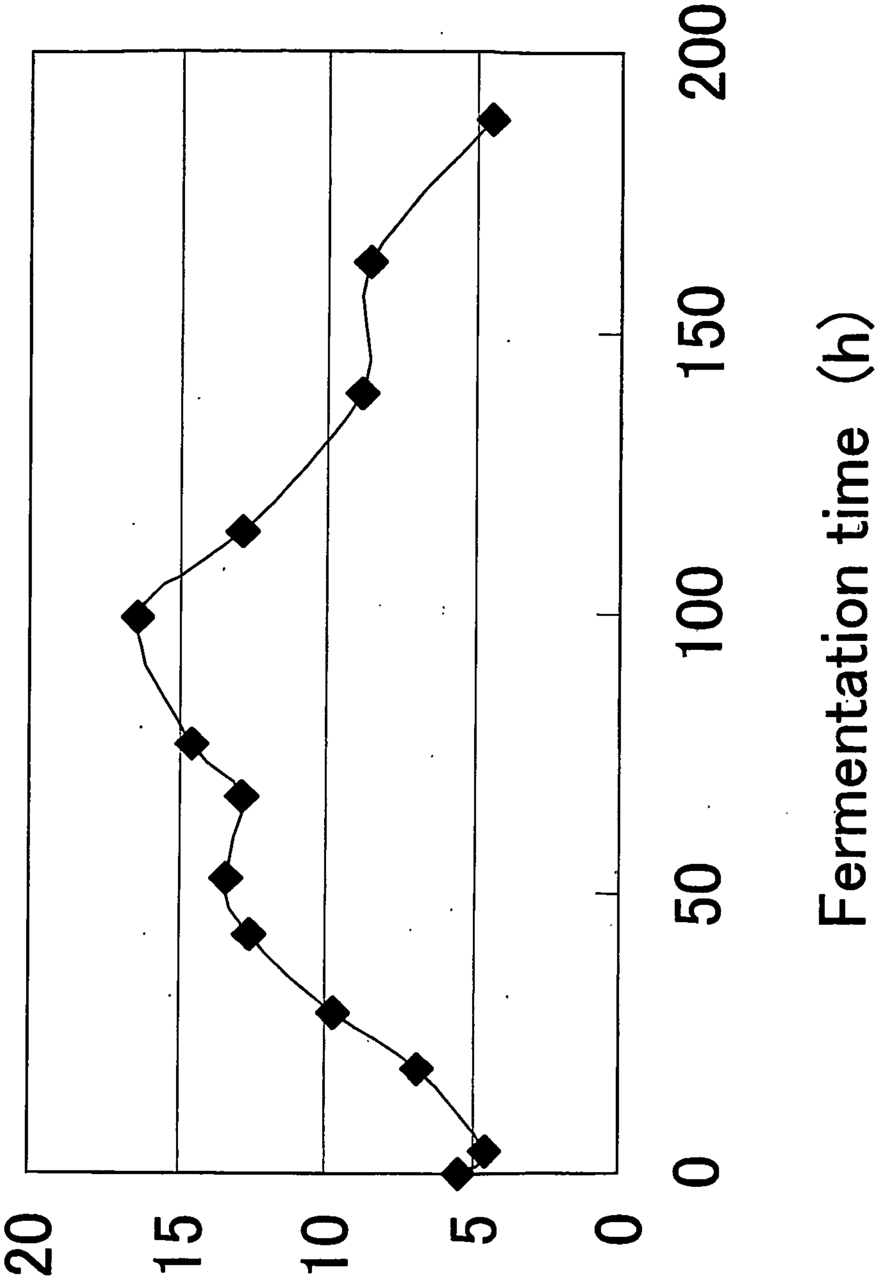

FIG. 2

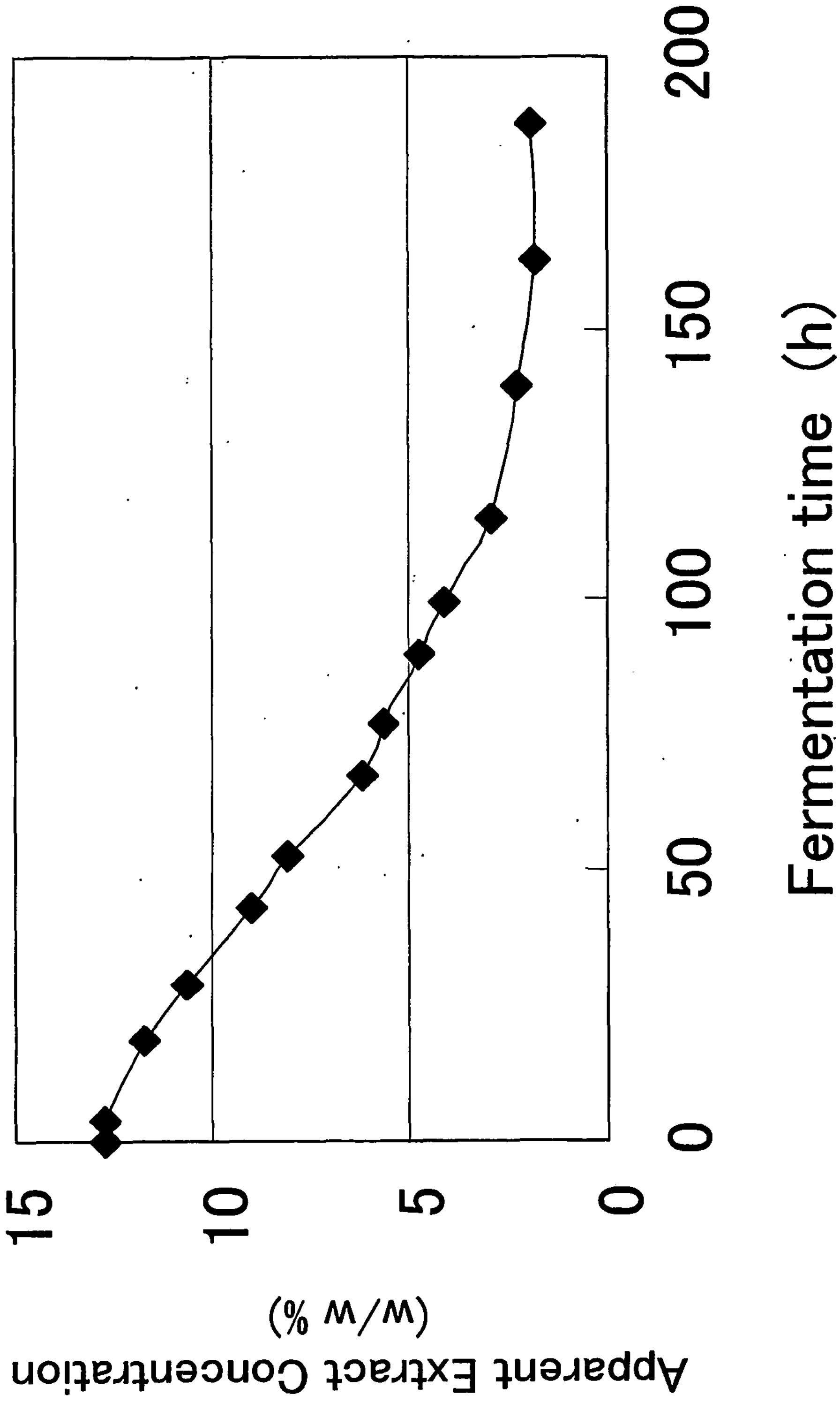

Apparent Extract Concentration (w/w %)
15
10
5
0
0
50
100
150
200
Fermentation time (h)

FIG. 3

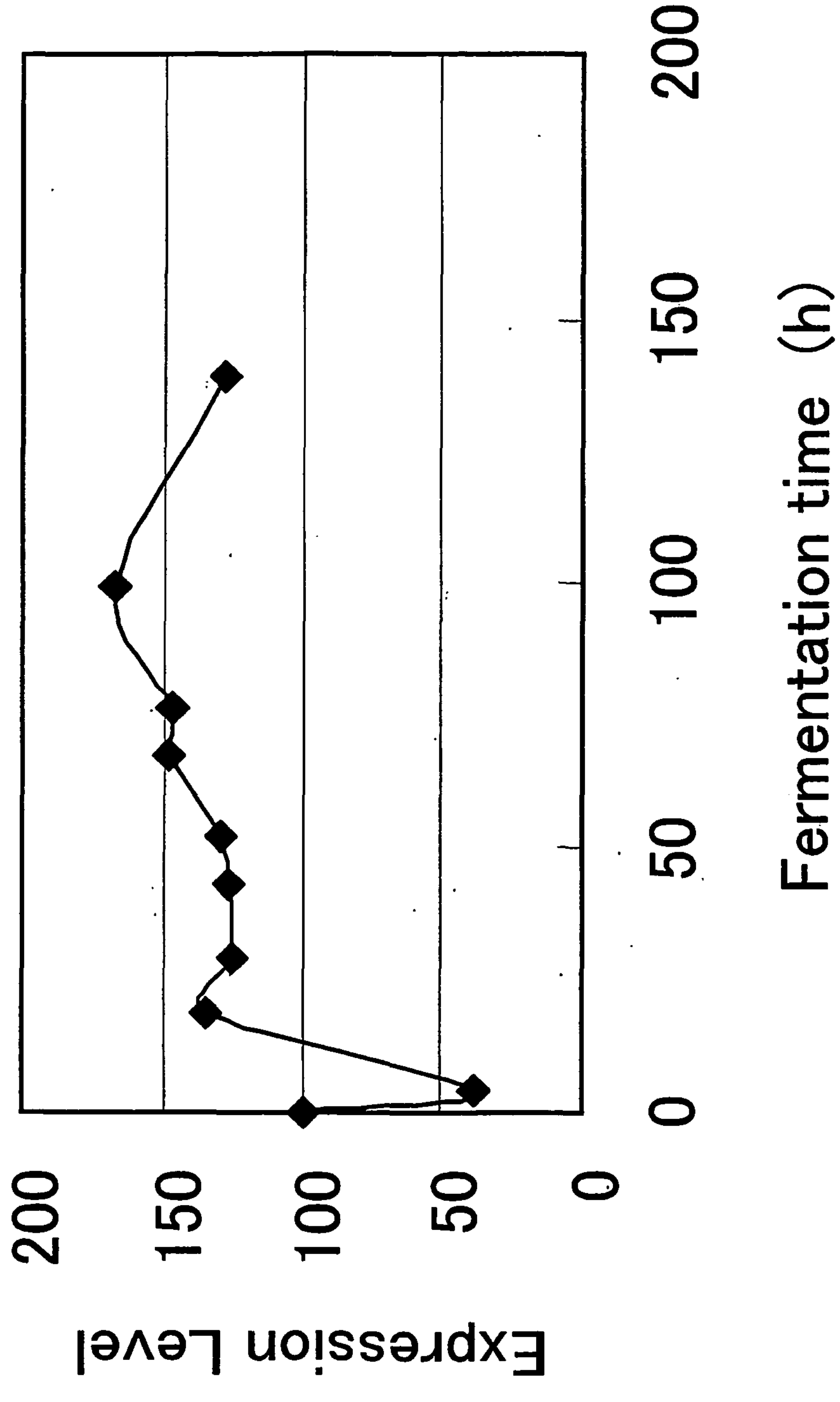
Expression Level
200
150
100
50
0
0
50
100
150
200
Fermentation time (h)

FIG. 4

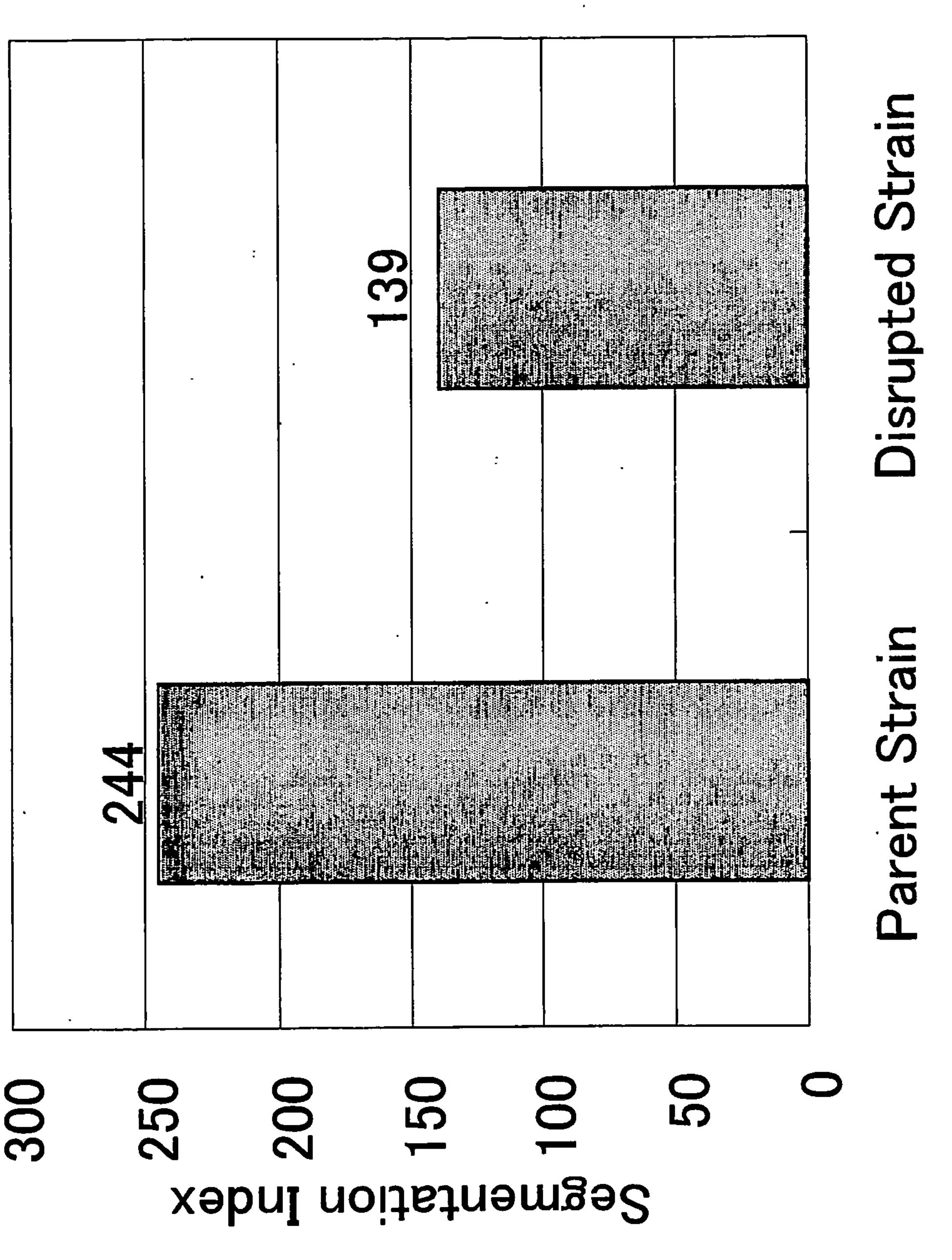
300
250
200
150
100
50
0
Segmentation Index
244
139
Parent Strain
Disrupted Strain

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H636734 B
- JP 3044284 B
- JP 3643404 B
- WO 01040514 A
- JP 2004283169 A
- JP H8205890 B
- JP 2002516062 PCT
- US 2002086356 A
- JP 6253826 A
- JP H8205900 B
- JP HB205890 B
- JP H07303475 B
- JP 2006049135 A

### Non-patent literature cited in the description


- **BIDARD et al.** *YEAST,* 1995, vol. 11 (9), 809
- MOLECULAR CLONING 3rd Ed., CURRENT PROTOCOLS IN MOLECULAR BIOLOGY. John Wiley & Sons, 1987
- **KARLIN ; ALTSCHUL.** *Proc. Natl. Acad. Sci. USA,* 1990, vol. 87, 2264-2268
- *Proc. Natl. Acad. Sci. USA,* 1993, vol. 90, 5873
- **ALTSCHUL SF et al.** *J. Mol. Biol.,* 1990, vol. 215, 403
- **HIRAJIMA ; INOUE.** New Biochemistry Experiment Course 2 Nucleic Acids IV Gene Replication and Expression. Tokyo Kagaku Dozin Co., Ltd, 1993, 319-347
- *Nature Genetics,* February 2000, vol. 24 (2), 180-183
- *Genesis,* April 2000, vol. 26 (4), 240-244
- *Nature,* 21 September 2002, vol. 407 (6802), 319-20
- *Genes & Dev.,* 15 April 2002, vol. 16 (8), 948-958
- *Proc. NatL Acad. Sci. USA.,* 16 April 2002, vol. 99 (8), 5515-5520
- *Science,* 19 April 2002, vol. 296 (5567), 550-553
- *Proc Natl. Acad. Sci. USA,* 30 April 2002, vol. 99 (9), 6047-6052
- *Nature Biotechnology,* May 2002, vol. 20 (5), 497-500
- *Nature Biotechnology,* May 2002, vol. 20 (5), 500-505
- *Nucleic Acids Res.,* 15 May 2002, vol. 30 (10), e46
- *FEBS Lett.,* 1988, vol. 228, 228
- *FEBS Lett.,* 1988, vol. 239, 285
- *Nucl. Acids. Res.,* 1989, vol. 17, 7059
- *Nature,* 1986, vol. 323, 349
- *NucL Acids. Res.,* 1991, vol. 19, 6751
- *Protein Eng,* 1990, vol. 3, 733
- *NucL Acids Res.,* 1991, vol. 19, 3875
- *Nucl. Acids Res.,* 1991, vol. 19, 5125
- *Biochem Biophys Res Commun,* 1992, vol. 186, 1271
- **SMYTH DR.** *Curr. Biol.,* 1997, vol. 7, R793
- **MARTIENSSEN R.** *Curr. Biol.,* 1996, vol. 6, 810
- MOLECULAR CLONING 3rd Ed., CURRENT PROTOCOLS IN MOLECULAR BIOLOGY. Nuc. Acids. Res. 1982, vol. 10, 6487
- *Proc. Natl. Acad. Sci. USA,* 1982, vol. 79, 6409
- *Gene,* 1985, vol. 34, 315
- *Nuc. Acids. Res.,* 1985, vol. 13, 4431
- *Proc. Natl. Acad Sci. USA,* 1985, vol. 82, 488
- *Proc. Natl. Acad. Sci. USA,* 1979, vol. 76, 4951
- *Methods in Enzymology,* 1983, vol. 101, 202
- *Nucleic Acids Res.,* 2001, vol. 29, 4238-4250
- *Appl Environ Microbiol.,* 2006, vol. 72, 5266-5273
- **J. R BROACH et al.** EXPERIMENTAL MANIPULATION OF GENE EXPRESSION. Academic Press, 1983, vol. 83
- **M. D. ROSE et al.** *Gene,* 1987, vol. 60, 237
- **K. STRUHL et al.** *Proc. Natl. Acad Sci. USA,* 1979, vol. 76, 1035
- **M. F. TUITE et al.** *EMBO J,* 1982, vol. 1, 603
- **MARIN et al.** *Proc. Natl. Acad. Sci. USA,* 1984, vol. 81, 337
- **JUNJI INOKOSHI et al.** *Biochemistry,* 1992, vol. 64, 660
- **HUSSAIN et al.** *Gene,* 1991, vol. 101, 149
- *Meth. Enzym.,* 1990, vol. 194, 182
- *Proc. Natl. Acad Sci. USA,* 1978, vol. 75, 1929
- *J. Bacteriology,* 1983, vol. 153, 163
- *Proc. Natl. Acad. Sci. USA,* 1978, vol. 75, 1929
- METHODS IN YEAST GENETICS. A Cold Spring Harbor Laboratory Course Manual

- Genetic Engineering. Plenum Press, 1979, vol. 1, 117
- MOLECULAR CLONING 3rd Ed., and METHODS IN YEAST GENETICS, A LABORATORY MANUAL. Cold Spring Harbor Laboratory Press
- METHODS IN YEAST GENETICS. Cold Spring Harbor Laboratory Press, 1990, vol. 130
- CURRENT PROTOCOLS IN MOLECULAR BIOLOGY. John Wiley & Sons, 1994
- BIOCHEMISTRY EXPERIMENTS. Yeas Molecular Genetic Experiments. vol. 39, 67-75
- **F. SANGER.** *Science,* 1981, vol. 214, 1215
- **GOLDSTEIN et al.** *Yeast,* 1999, vol. 15, 1541

SEQUENCE LISTING

<110> Suntory Limited

<120> Gene encoding protein responsible for flocculation property of yeast and use thereof

<130> PCT06-0133

<150> JP2006-49135
<151> 2006-02-24

<160> 6

<170> PatentIn version 3.3

<210> 1
<211> 4074
<212> DNA
<213> Saccharomyces sp.

<400> 1

```
atggattcca aggacacgca aaagttgctt aaggagcacc gaattccctc catcgatgtc      60
ggctggctgg tcaggcccaa cgcctccgcc ctcaggcacg agaggtccaa gtcgacggct     120
gagagcgtgg cccccgacgt ccagatggac actgctcact cgtccgtctt cgacaagtct     180
gtagacacgt cttgcggcat tctctcccca aatgataggg tgcgccgcca ctccgtggcg     240
gcggccgctc tgatgaataa ccagcacatc actgttgccc ctaccgcgcc ctccacgaac     300
ttcccctccg gtagaggacg gtccaaatcc gtggtagaga tcacgatgcc aggctccggc     360
gttgacgccg atgctgacgc tggacttcgc catcaccatc accatcatca ccatcatgcg     420
gaagacgcag cttcgtcacc tgcccccaag aagatcggtt tcttcaagag tttatttggc     480
aaaaggaagg gagaacaaga gcagcagaag cgcgaaaaag acaaggagga acaagaacaa     540
aaccgctcgc cctccccggg ccacgtgaac cgcaactcca taagaagaga acgaaccgcg     600
accatttctg ctgagtcacc tccgcccttg cagtacgtgg ccccgccgtc gtacaacgac     660
accgtggtgc cgctgacgag gtccaagact gaatccgagg tgtactacga gaaccatccg     720
cagaacaaca ctcacggtca catccacacg caacacactt tcgatgaagg tcaaactgat     780
tgcagctccc ccatagatgg cgccgcctgc agcggcactc gtcctgaccc gagattgatg     840
gatttcttgc ggtactacaa atctaaggac tacaaattag ccgcctttaa agaggggaac     900
ttcctgcaat ccacgccctc gtcgccgatc aagagaaaca cacgggcctc gttttccctt     960
cagaatgaca gacaattgcc ccccaagtta gccgcccgcc agaaattcga cgttaagggc    1020
```

```
agacctatcc cgccgcatcc ggacaaaccg aggttggcgc ccgccttcaa gaagaagaag 1080
caaccagtcg ctatggcatc gttggaggcc gtggactcta actcagacgt ctcgtcctcc 1140
gcgcagaaca acaacgcggc gccgtcttct cacaagttcg gcgcatttct cagaaaggtt 1200
acctcttacg ggaacaacgc ctcatcctcc tccgccaata ccaacaacct gctcaatgcc 1260
tcgtccacgt ccgtatggtc tgccaactcg ctcgagttcg atcctgcaaa gataaccgtc 1320
gtgcccgggc ttgagtacgt caagcccttg aaacacgtct cctttgctac gaacacctac 1380
ttcaacgacc ctccacagca gatctgtagt aaaaacccca gaagaggcga agtggaggtg 1440
gagcccaacg ggtccgtcgt tatacacaga ctgacaccgc aagagaggaa gaaaattatg 1500
gagtcgacaa gcctgggcgt cgtcgttggg ggcactggcc aattgaaact gctaaatcca 1560
gaagaggacg acatcaacgc caagagcaaa gaggagatgg ctccgcaaag gcaaaacgca 1620
gccgaagcgc ccgatgagga agatggcaac ccgcagagaa gaaacatcgt tatggctgcc 1680
gcagaagccg ccgcagaagc cagagccaaa gaggccccca acgagttgaa gcgtattgtc 1740
acgaacaatg aggaggaagt catcgttagt aagaccgcgt gtcatttaac cattgataag 1800
ccgatgatct caagaagggg tgcttccaca acctctctgg cctccatggt gtcgagtgat 1860
actaatggta cgaacgcaga tgacgagaag gagaccatgc ccccacccaa ccaaaagatc 1920
ccccacgata tcgtgtatac ccgctgctgt catttaagag aaattttgcc aataccggcc 1980
actttgaaac agttgaaaaa gggatccacc gaccccatcc ccatcttaca attgcgtaac 2040
ccaagaccct ccatggtgga aatttggtca tttagcgact tcttgagtgt ggcccccgtt 2100
ttgtgtcttt cgttggacgg tgtgcaatta acggtgcaaa tgctcagaag cattttgagt 2160
tctttgattt acaacccaca tttccaaaaa ctgtccttga gaaacacttt cctggatgaa 2220
gaaggctgga aatgttatgg ctatttgtc tccaaggcca ggtctttgca ttccatcgat 2280
ttgaccatgg tcccctccat caagacaaat gtacagaagc cctccaaatc ctctttgaag 2340
tcaaccatcc tcagaatgca gtgcaataca gacgacaggt ccgacatgaa ttgggatctc 2400
ttgactgctt ccatcgccct tagaggcggt ttggacgaaa ttgtcatttc gggggcaaaa 2460
atgaatggag cacaattcaa aaattttatc cttgtcgctt gtatcgctac cgaaagactg 2520
ggcctggctt ataatggctt gtccaagagt caatgtgacg atctggccag ttgggtggtg 2580
caatccgaag tgacaggttt ggatatcgga ttcaatgatc tgaatgggaa actatcgtcc 2640
ttcactaatg ctgtcctggg caaaattcaa aaggtcaacg agaaaaacgt cttcaagttt 2700
ctatctttaa atggaacaaa tttagaagta aaggaacacg atacttatga aaacaacgag 2760
gttttgaaat tgatcagtgt cctgtgctat tcagaaaacc tgaagttctt agatatctcg 2820
aacaaccctg caatctttcc gtattgtgtc ccgacactaa ttgattttct gcccgttttc 2880
gtcaacttgg tccgtttaca aatcgactac aataatttaa gctccaccag tgtggttatg 2940
ttggctgaag tattgcccat gtgttcgaga ctgagttact tctccatgtt ggggacagaa 3000
ttggatctgg catcctctaa agccctagcg gaagccgtga gaaagagctc atcgttgatg 3060
accttggacg tggattacgc ttacatgcca gagaatatca aggaaaaaat ttcactatat 3120
gccatgagaa acattcaagg tgaattgaaa agagttcaaa acgacgacaa agatatcaaa 3180
gacactcaat tttcaagttt acaagaccaa ctatcgcttt tattaactga aaaggcagac 3240
gattccgaac actataaaaa aatggtcgaa aacttcatgg ccaaaatcgc attggcccgt 3300
atcaagatca gtaaagtggt tcacgacctt ttcggtttaa aacttaatgg tcaattaaat 3360
```

```
ttggaaggca aagaagcttt gattagattg tgtttcatcg aggcaagttt ggaaagaggt    3420
tccgacttat taagacaaag acatcataat gctttgaaac cacgaagctc attctcggca    3480
aaccaaacag acgcaaattc agaaagttgt caaagaatgt tattgagttc atctatccta    3540
caaaattcag accacattgc cctgatgcca tttggcagtg cactagtcga aaaatctagc    3600
gcggacgcag aagacgctgt agagtttaga gaaggcgatg attcaaatac caaccatgaa    3660
gagggcccag ctaatgatct acaagcccaa gatcaagtcg atattaagaa taaatactct    3720
atcatcaaaa aagaactaga acacgaaaag cttgttggcg gtggggattt accagtggac    3780
aaagacattc taaatcgcgc cgctcaatcc cttgatagtg atcaaatcaa agagtttttg    3840
ttgaaaaatg acgtttctac tatcttgggt gttattgacg aattgcatag ccagggatat    3900
catttgcatc atattttcaa aaagcaggga aaccaatctg ataaacaaga agacgctacc    3960
cttcaagcga aggatgcgcc agaccagtcc caagcagaca attcaagtga taacatatct    4020
cttacgaccg accaaacatc gacagggttc tgccagtgcc aagacgaaca atga          4074
```

<210> 2
<211> 1357
<212> PRT
<213> Saccharomyces sp.

<400> 2

```
Met Asp Ser Lys Asp Thr Gln Lys Leu Leu Lys Glu His Arg Ile Pro
1               5                   10                  15
Ser Ile Asp Val Gly Trp Leu Val Arg Pro Asn Ala Ser Ala Leu Arg
            20                  25                  30
His Glu Arg Ser Lys Ser Thr Ala Glu Ser Val Ala Pro Asp Val Gln
        35                  40                  45
Met Asp Thr Ala His Ser Ser Val Phe Asp Lys Ser Val Asp Thr Ser
    50                  55                  60
Cys Gly Ile Leu Ser Pro Asn Asp Arg Val Arg Arg His Ser Val Ala
65                  70                  75                  80
Ala Ala Ala Leu Met Asn Asn Gln His Ile Thr Val Ala Pro Thr Ala
                85                  90                  95
Pro Ser Thr Asn Phe Pro Ser Gly Arg Gly Arg Ser Lys Ser Val Val
            100                 105                 110
Glu Ile Thr Met Pro Gly Ser Gly Val Asp Ala Asp Ala Asp Ala Gly
        115                 120                 125
Leu Arg His His His His His His His His His Ala Glu Asp Ala Ala
    130                 135                 140
Ser Ser Pro Ala Pro Lys Lys Ile Gly Phe Phe Lys Ser Leu Phe Gly
145                 150                 155                 160
```

Lys Arg Lys Gly Glu Gln Glu Gln Gln Lys Arg Glu Lys Asp Lys Glu
165 170 175
Glu Gln Glu Gln Asn Arg Ser Pro Ser Pro Gly His Val Asn Arg Asn
180 185 190
Ser Ile Arg Arg Glu Arg Thr Ala Thr Ile Ser Ala Glu Ser Pro Pro
195 200 205
Pro Leu Gln Tyr Val Ala Pro Pro Ser Tyr Asn Asp Thr Val Val Pro
210 215 220
Leu Thr Arg Ser Lys Thr Glu Ser Glu Val Tyr Tyr Glu Asn His Pro
225 230 235 240
Gln Asn Asn Thr His Gly His Ile His Thr Gln His Thr Phe Asp Glu
245 250 255
Gly Gln Thr Asp Cys Ser Ser Pro Ile Asp Gly Ala Ala Cys Ser Gly
260 265 270
Thr Arg Pro Asp Pro Arg Leu Met Asp Phe Leu Arg Tyr Tyr Lys Ser
275 280 285
Lys Asp Tyr Lys Leu Ala Ala Phe Lys Glu Gly Asn Phe Leu Gln Ser
290 295 300
Thr Pro Ser Ser Pro Ile Lys Arg Asn Thr Arg Ala Ser Phe Ser Leu
305 310 315 320
Gln Asn Asp Arg Gln Leu Pro Pro Lys Leu Ala Ala Arg Gln Lys Phe
325 330 335
Asp Val Lys Gly Arg Pro Ile Pro Pro His Pro Asp Lys Pro Arg Leu
340 345 350
Ala Pro Ala Phe Lys Lys Lys Lys Gln Pro Val Ala Met Ala Ser Leu
355 360 365
Glu Ala Val Asp Ser Asn Ser Asp Val Ser Ser Ser Ala Gln Asn Asn
370 375 380
Asn Ala Ala Pro Ser Ser His Lys Phe Gly Ala Phe Leu Arg Lys Val
385 390 395 400
Thr Ser Tyr Gly Asn Asn Ala Ser Ser Ser Ser Ala Asn Thr Asn Asn
405 410 415
Leu Leu Asn Ala Ser Ser Thr Ser Val Trp Ser Ala Asn Ser Leu Glu
420 425 430
Phe Asp Pro Ala Lys Ile Thr Val Val Pro Gly Leu Glu Tyr Val Lys
435 440 445
Pro Leu Lys His Val Ser Phe Ala Thr Asn Thr Tyr Phe Asn Asp Pro
450 455 460
Pro Gln Gln Ile Cys Ser Lys Asn Pro Arg Arg Gly Glu Val Glu Val

465 470 475 480

Glu Pro Asn Gly Ser Val Val Ile His Arg Leu Thr Pro Gln Glu Arg

485 490 495

Lys Lys Ile Met Glu Ser Thr Ser Leu Gly Val Val Val Gly Gly Thr

500 505 510

Gly Gln Leu Lys Leu Leu Asn Pro Glu Glu Asp Asp Ile Asn Ala Lys

515 520 525

Ser Lys Glu Glu Met Ala Pro Gln Arg Gln Asn Ala Ala Glu Ala Pro

530 535 540

Asp Glu Glu Asp Gly Asn Pro Gln Arg Arg Asn Ile Val Met Ala Ala

545 550 555 560

Ala Glu Ala Ala Ala Glu Ala Arg Ala Lys Glu Ala Pro Asn Glu Leu

565 570 575

Lys Arg Ile Val Thr Asn Asn Glu Glu Glu Val Ile Val Ser Lys Thr

580 585 590

Ala Cys His Leu Thr Ile Asp Lys Pro Met Ile Ser Arg Arg Gly Ala

595 600 605

Ser Thr Thr Ser Leu Ala Ser Met Val Ser Ser Asp Thr Asn Gly Thr

610 615 620

Asn Ala Asp Asp Glu Lys Glu Thr Met Pro Pro Pro Asn Gln Lys Ile

625 630 635 640

Pro His Asp Ile Val Tyr Thr Arg Cys Cys His Leu Arg Glu Ile Leu

645 650 655

Pro Ile Pro Ala Thr Leu Lys Gln Leu Lys Lys Gly Ser Thr Asp Pro

660 665 670

Ile Pro Ile Leu Gln Leu Arg Asn Pro Arg Pro Ser Met Val Glu Ile

675 680 685

Trp Ser Phe Ser Asp Phe Leu Ser Val Ala Pro Val Leu Cys Leu Ser

690 695 700

Leu Asp Gly Val Gln Leu Thr Val Gln Met Leu Arg Ser Ile Leu Ser

705 710 715 720

Ser Leu Ile Tyr Asn Pro His Phe Gln Lys Leu Ser Leu Arg Asn Thr

725 730 735

Phe Leu Asp Glu Glu Gly Trp Lys Met Leu Cys Tyr Phe Val Ser Lys

740 745 750

Ala Arg Ser Leu His Ser Ile Asp Leu Thr Met Val Pro Ser Ile Lys

755 760 765

Thr Asn Val Gln Lys Pro Ser Lys Ser Ser Leu Lys Ser Thr Ile Leu

770 775 780

Arg Met Gln Cys Asn Thr Asp Asp Arg Ser Asp Met Asn Trp Asp Leu
785 790 795 800
Leu Thr Ala Ser Ile Ala Leu Arg Gly Gly Leu Asp Glu Ile Val Ile
805 810 815
Ser Gly Ala Lys Met Asn Gly Ala Gln Phe Lys Asn Phe Ile Leu Val
820 825 830
Ala Cys Ile Ala Thr Glu Arg Leu Gly Leu Ala Tyr Asn Gly Leu Ser
835 840 845
Lys Ser Gln Cys Asp Asp Leu Ala Ser Trp Val Val Gln Ser Glu Val
850 855 860
Thr Gly Leu Asp Ile Gly Phe Asn Asp Leu Asn Gly Lys Leu Ser Ser
865 870 875 880
Phe Thr Asn Ala Val Leu Gly Lys Ile Gln Lys Val Asn Glu Lys Asn
885 890 895
Val Phe Lys Phe Leu Ser Leu Asn Gly Thr Asn Leu Glu Val Lys Glu
900 905 910
His Asp Thr Tyr Glu Asn Asn Glu Val Leu Lys Leu Ile Ser Val Leu
915 920 925
Cys Tyr Ser Glu Asn Leu Lys Phe Leu Asp Ile Ser Asn Asn Pro Ala
930 935 940
Ile Phe Pro Tyr Cys Val Pro Thr Leu Ile Asp Phe Leu Pro Val Phe
945 950 955 960
Val Asn Leu Val Arg Leu Gln Ile Asp Tyr Asn Asn Leu Ser Ser Thr
965 970 975
Ser Val Val Met Leu Ala Glu Val Leu Pro Met Cys Ser Arg Leu Ser
980 985 990
Tyr Phe Ser Met Leu Gly Thr Glu Leu Asp Leu Ala Ser Ser Lys Ala
995 1000 1005
Leu Ala Glu Ala Val Arg Lys Ser Ser Ser Leu Met Thr Leu Asp
1010 1015 1020
Val Asp Tyr Ala Tyr Met Pro Glu Asn Ile Lys Glu Lys Ile Ser
1025 1030 1035
Leu Tyr Ala Met Arg Asn Ile Gln Gly Glu Leu Lys Arg Val Gln
1040 1045 1050
Asn Asp Asp Lys Asp Ile Lys Asp Thr Gln Phe Ser Ser Leu Gln
1055 1060 1065
Asp Gln Leu Ser Leu Leu Leu Thr Glu Lys Ala Asp Asp Ser Glu
1070 1075 1080
His Tyr Lys Lys Met Val Glu Asn Phe Met Ala Lys Ile Ala Leu

1085 1090 1095
Ala Arg Ile Lys Ile Ser Lys Val Val His Asp Leu Phe Gly Leu
1100 1105 1110
Lys Leu Asn Gly Gln Leu Asn Leu Glu Gly Lys Glu Ala Leu Ile
1115 1120 1125
Arg Leu Cys Phe Ile Glu Ala Ser Leu Glu Arg Gly Ser Asp Leu
1130 1135 1140
Leu Arg Gln Arg His His Asn Ala Leu Lys Pro Arg Ser Ser Phe
1145 1150 1155
Ser Ala Asn Gln Thr Asp Ala Asn Ser Glu Ser Cys Gln Arg Met
1160 1165 1170
Leu Leu Ser Ser Ser Ile Leu Gln Asn Ser Asp His Ile Ala Leu
1175 1180 1185
Met Pro Phe Gly Ser Ala Leu Val Glu Lys Ser Ser Ala Asp Ala
1190 1195 1200
Glu Asp Ala Val Glu Phe Arg Glu Gly Asp Asp Ser Asn Thr Asn
1205 1210 1215
His Glu Glu Gly Pro Ala Asn Asp Leu Gln Ala Gln Asp Gln Val
1220 1225 1230
Asp Ile Lys Asn Lys Tyr Ser Ile Ile Lys Lys Glu Leu Glu His
1235 1240 1245
Glu Lys Leu Val Gly Gly Gly Asp Leu Pro Val Asp Lys Asp Ile
1250 1255 1260
Leu Asn Arg Ala Ala Gln Ser Leu Asp Ser Asp Gln Ile Lys Glu
1265 1270 1275
Phe Leu Leu Lys Asn Asp Val Ser Thr Ile Leu Gly Val Ile Asp
1280 1285 1290
Glu Leu His Ser Gln Gly Tyr His Leu His His Ile Phe Lys Lys
1295 1300 1305
Gln Gly Asn Gln Ser Asp Lys Gln Glu Asp Ala Thr Leu Gln Ala
1310 1315 1320
Lys Asp Ala Pro Asp Gln Ser Gln Ala Asp Asn Ser Ser Asp Asn
1325 1330 1335
Ile Ser Leu Thr Thr Asp Gln Thr Ser Thr Gly Phe Cys Gln Cys
1340 1345 1350
Gln Asp Glu Gln
1355

<210> 3
<211> 40
<212> DNA
<213> Artificial

<220>
<223> Primer

<400> 3
gagctcatag cggccatgga ttccaaggac acgcaaaagt 40

<210> 4
<211> 42
<212> DNA
<213> Artificial

<220>
<223> Primer

<400> 4
ggatcctatg cggccgctac ataaaattat catacataaa ag 42

<210> 5
<211> 70
<212> DNA
<213> Artificial Sequence

<220>
<223> Primer

<400> 5

cttaacgcac atatatacgc aaatacacat catagctcca caactgctcc ccttgacagt 60
cttgacgtgc 70

<210> 6
<211> 70
<212> DNA
<213> Artificial Sequence

<220>
<223> Primer

<400> 6

atgcaacatg atctctaaac tccaatgaaa agaaagaaag aaatccttca cgcacttaac 60
ttcgcatctg 70